# EUROPÄISCHE PATENTSCHRIFT

(11) **EP 2 420 212 B1**
(45) Veröffentlichungstag und Bekanntmachung des Hinweises auf die Patenterteilung: **02.03.2016**
(21) Anmeldenummer: 11175493.3
(22) Anmeldetag: 27.07.2011
(51) Int. Cl.: A61F 2/66, A61F 2/50

(54) **Fußprothese**
Foot prosthesis
Prothèse du pied

(30) Priorität: 19.08.2010 DE 102010034893
(43) Veröffentlichungstag der Anmeldung: 22.02.2012
(73) Patentinhaber: medi GmbH & Co. KG, 95448 Bayreuth (DE)
(72) Erfinder:
(74) Vertreter: Blaumeier, Jörg

(56) Entgegenhaltungen:
- WO-A2-02/30340
- DE-A1- 4 037 928
- US-A1- 2005 038 524
- US-A1- 2008 033 578

## Beschreibung

Die Erfindung betrifft eine Fußprothese, umfassend ein Oberteil und ein beim Laufen bodenseitig aufsetzendes Unterteil, wobei das Oberteil und das Unterteil über ein im Fersenbereich angeordnetes elastisches Dämpfungselement miteinander verbunden sind und das Oberteil und das Unterteil übereinander und voneinander beabstandet in den Bereich der Fußspitze laufen. DE 40 37 928 A stellt der nächste Stand der Technik dar.

Eine Fußprothese der beschriebenen Art ermöglicht dem Träger ein komfortables, über das Dämpfungselement gedämpftes Auftreten und ein dem realen Fuß angenähertes Laufen. Moderne Fußprothesen bestehen beispielsweise aus einem Karbonlaminat, also einem Kohlefaserverbundwerkstoff. Sie weisen ein Oberteil auf, über das die Fußprothese mit einem Verbindungsschaft oder ähnlichem, mit dem die gesamte Prothese am Beinstumpf befestigt wird, über ein geeignetes Verbindungsmittel gekoppelt wird. Das Oberteil weist einen näherungsweise horizontal verlaufenden Abschnitt auf, der sich vom Fersenbereich bis zum Fußspitzenbereich erstreckt. Im Bereich der Fußspitze ist das Oberteil mit einem Unterteil verbunden, das beim Laufen bodenseitig aufsetzt. Oberteil und Unterteil sind voneinander beabstandet, liegen jedoch näherungsweise deckungsgleich übereinander. Zwischen beiden befindet sich im Fersenbereich ein elastisches Dämpfungselement, beispielsweise aus einem gummiartigen Material, das als Auftrittsdämpfer wirkt und die beim Aufsetzen wirkende Kraft dämpft. Im Bereich zwischen Dämpfungselement und Fußspitze bilden das Ober- und Unterteil eine Feder, die beim Laufen gebogen und beansprucht wird. Bei einer Laufbewegung setzt die Fußprothese mit dem hinteren Ende des Unterteils am Boden auf. Das Fersenelastomer wirkt als Auftrittsdämpfer und Drehpunkt zwischen Rückfuß- und Vorderfußhebel, also zwischen Fersenbereich und Fußspitzenbereich. Der Rückfußhebel wird durch die Bodenreaktionskraft nach oben vorgespannt, was ein Drehmoment im Dämpfungselement zur Folge hat. Dadurch wird der Vorderfußbereich, also der Bereich zwischen Dämpfungselement und Fußspitze, vorgespannt, so dass sich dieser Abschnitt weitet, das heißt, dass sich in diesem Bereich das Oberteil vom Unterteil entfernt, es kommt zu einer Aufbiegung. Auf diese Weise wird dort Energie gespeichert. Diese Energie wird bei Bodenkontakt des Vorderfußes, wenn also auch die Fußspitze aufsetzt, wieder frei und erleichtert so die Vorwärtsbewegung. Beim Übergang in die frühe Standphase wandert der Vektor der Bodenreaktionskraft, der bisher hinter dem Dämpfungselement, also im hinteren Fersenbereich lag, vor das Dämpfungselement. Hierdurch entsteht ein Moment im Dämpfungselement als Drehpunkt, das den unteren und den oberen Bereich des Vorderfußes zusammendrückt. Das heißt, dass in Folge der Gewichtsverlagerung die vorherige Aufweitung zwischen Oberteil und Unterteil wieder abgebaut wird. Dieses Moment wird im Verlauf des Abrollvorgangs größer, da der Vektor der Bodenreaktionskraft immer weiter nach vorne wandert, wodurch sich die beiden Bereiche des Oberteils und des Unterteils, die zuvor auseinander gespannt waren, einander immer weiter annähern, bis sie zuerst linienförmig und später flächig in Kontakt zueinander kommen. Ab diesem Zeitpunkt, wenn es zur Berührung kommt, steigt der Vorderfußwiderstand überproportional. Das heißt, dass folglich einer etwaigen elastischen Fußverformung ein deutlich höherer Widerstand entgegengesetzt wird, resultierend aus den einander berührenden Bereichen des Oberteils und des Unterteils im Bereich zwischen Dämpfungselement und Fußspitze.

Die grundsätzliche Härte der Fußprothese, also letztlich ihre Biegefähigkeit im Bereich der Feder und Abrollfähigkeit, wird bei modernen Prothesen aus Karbonmaterial letztlich durch die jeweilige Auslegung der Oberteil- und Unterteilhärte, also letztlich des Teilematerials selbst eingestellt, das heißt, dass je nach gewünschter Härte das die Prothese bildende Karbonmaterial entsprechend dicker ausgeführt ist oder vom Verbund her entsprechend härter eingestellt ist etc. Eine Möglichkeit zur Einstellung der Härte ist dem Orthopädietechniker oder dem Benutzer nicht gegeben.

Der Erfindung liegt damit das Problem zugrunde, eine Fußprothese mit der Möglichkeit zur Einstellung der Härte anzugeben.

Zur Lösung des Problems ist bei einer Fußprothese der eingangs genannten Art erfindungsgemäß vorgesehen, dass im Bereich zwischen dem Dämpfungselement und der Fußspitze zwischen das Ober- und das Unterteil wenigstens ein Füllstück eingebracht ist, über welches die Biegesteifigkeit des beim Laufen über das Füllelement gekoppelten Ober- und Unterteils erhöht wird, wobei das Füllstück ein flächiges Bauteil mit wenigstens einer anhaftender Anlagefläche ist.

Die erfindungsgemäße Fußprothese zeichnet sich dadurch aus, dass in den Bereich zwischen Dämpfungselement und Fußspitze, also in den dort realisierten Hohlraum zwischen Oberteil und Unterteil, ein Füllstück eingesetzt wird. Dieses tritt während der Laufbewegung in Kontakt mit einer oder mit beiden einander zugewandten Flächen des Ober- und Unterteils, je nach Auslegung des Füllstücks. Hierdurch kommt es folglich zu einer Veränderung des Anlagevorgangs oder Koppelvorgangs des Oberteils mit dem Unterteil, die erfindungsgemäß über das Füllstück gekoppelt werden. Der sich beim Laufen ergebenden Bewegung des Oberteils und relativ zum Unterteil wird über das Füllstück, an dem temporär beide anliegen, ein Widerstand entgegengesetzt, so dass es zu einer Versteifung der aus Oberteil und Unterteil im Bereich zwischen Dämpfungselement und Fußspitze gebildeten Feder kommt. Das heißt, dass eine steifigkeitserhöhende Verbindung nicht erst dann während der Laufbewegung einsetzt, wenn das Oberteil das Unterteil kontaktiert, sondern bereits wesentlich früher, nämlich dann, wenn ein Flächenkontakt über das Füllstück einsetzt. Da dieses im Bereich zwischen Ober- und Unterteil angeordnet ist, kommt es zwangsläufig wesentlich früher zu einer flächigen Kontaktierung, im Vergleich mit einer Fußprothese ohne eingesetztem Füllstück. Das heißt, dass sich deutlich früher und daraus resultierend eben zu individuell einstellbaren Zeitpunkten eine Veränderung der Biegesteifigkeit ergibt. Infolge der Berührung kommt es auch zu einer Veränderung der einsetzenden Verschiebebewegung des Oberteils relativ zum Unterteil, die spätestens beim Ziehen oder Abdrücken einsetzt. Hierbei bewegen sich beide Teile etwa relativ zueiannder. Dieser Bewegung wird durch die Kopplung über das Füllelement ein Widerstand entgegengesetzt, der ebenfalles versteifend wirkt. Denn durch Integration des Füllstücks bedarf es einer vollständigen Annäherung von Oberteil zum Unterteil nicht mehr, vielmehr ergibt sich eine Widerstandserhöhung und damit eine Aufhärtung der Prothese bereits dann, wenn sich das Oberteil soweit dem Unterteil genähert hat, dass das Füllstück kontaktiert wird und folglich wegen der Flächenanlage eine nur noch gegen erhöhten Widerstand mögliche Einfederung und Relativverschiebung von Oberteil zum Unterteil möglich ist.

Das heißt, dass mit dem erfindungsgemäß zwischen oberem und unterem Bereich des Vorderfußes, also Oberteil und Unterteil integrierten Füllstück die Möglichkeit besteht, individuell einzustellen, ab welcher Phase des Schrittzyklus die Vorfußhärte überproportional anzusteigen beginnt. Damit bietet sich dem Orthopädietechniker die Möglichkeit, die Fußprothese individuell so anzupassen, dass es für den Träger am komfortabelsten ist, wobei sich selbstverständlich die Anpassung auch in Abhängigkeit der jeweiligen Nutzungssituation (Fußprothese für den Freizeitbereich, Fußprothese für den Sportbereich) individuell durchführen lässt.

Das Füllstück selbst ist bevorzugt ein flächiges Bauteil mit wenigstens einer anhaftenden Anlagefläche, das auf unterschiedliche Weise zwischen Oberteil und Unterteil gesetzt werden kann.

Nach einer ersten Erfindungsalternative kann das Füllstück fest mit dem Oberteil oder dem Unterteil verbunden sein, beispielsweise angeklebt sein. Das Füllstück wird gemäß dieser Alternative vom Orthopädietechniker nach individueller, trägerspezifischer Auswahl und gegebenenfalls Anpassung fest fixiert. Beim Laufen tritt es in Kontakt mit dem jeweils gegenüberliegenden Teil, dabei den Widerstand erzeugend. Ist also das Füllstück beispielsweise ein längliches flächiges Kunststoffbauteil aus einem hinreichend elastischem Elastomer, so wird es beispielsweise auf das Unterteil geklebt und tritt beim Laufen in Kontakt mit dem Oberteil. Der Umstand dass das Füllstück erst von Orthopädietechniker eingesetzt wird, ermöglicht die genaue Anpassung und Einstellung der Prothese bzw. deren Federverhalten auf die individuellen Bedürfnisse des Trägers.

Eine Alternative sieht vor, das Füllstück lösbar am Oberteil, am Unterteil oder am Dämpfungselement anzuordnen. Diese dem festen Verkleben alternative Anordnungsmöglichkeit bietet dem Orthopädietechniker oder Träger den besonderen Vorteil, das Füllstück bei Bedarf zu entnehmen oder gegen ein anderes Füllstück auszutauschen. Hierüber wird also ein Höchstmaß an Flexibilität hinsichtlich der individuellen Dämpfungs- respektive Härteauslegung der Fußprothese gegeben, da der Orthopädietechniker oder Träger nach Belieben die Protheseneinstellung variieren kann, indem er ein Füllstück entfernt oder gegen ein anderes, weicheres oder härteres oder längeres oder kürzeres Füllstück austauscht. Die grundsätzliche Auswahl und individuelle Anpassung des Füllstücks und damit die Einstellung der Prothese nimmt der Orthopädietechniker vor, die Vorteile der Austauschbarkeit bieten sich auch dem Träger.

Eine zweckmäßige Weiterbildung dieser Erfindungsalternative mit lösbarem Füllstück sieht vor, dass am Dämpfungselement ein erster Befestigungsabschnitt und am Füllstück ein zum ersten Befestigungsabschnitt komplementär geformter zweiter Befestigungsabschnitt vorgesehen ist, die lösbar miteinander in Eingriff bringbar sind. Gemäß dieser Ausgestaltung erfolgt also bevorzugt die Befestigung des Füllstücks am Dämpfungselement. Hierzu sind zwei komplementär zueinander ausgeführte Befestigungsabschnitte vorgesehen, einer am Dämpfungselement, einer am Füllstück, die zur Fixierung lösbar miteinander in Eingriff zu bringen sind.

Eine sichere Fixierung wird beispielsweise dadurch erreicht, dass der erste Befestigungsabschnitt eine zur Fußspitze hin offene Nut mit einem innenliegenden hinterschnittenen länglichen Haltevorsprung und der zweite Befestigungsabschnitt eine komplementär zur Nut und zum Haltevorsprung geformte Halteklaue, die den Haltevorsprung umgreift, ist. Die am Dämpfungselement vorgesehene Nut kann ohne weiteres hinreichend tief ausgeführt werden, da das Dämpfungselement selbst eine gewisse horizontale Länge aufweist. Durch die formkomplementäre Ausbildung der Nut nebst Haltevorsprung, beispielsweise als querschnittlich gesehen runde vorspringende Wulst ausgeführt, und der Halteklaue wird ein fester, formschlüssiger Halteverbund realisiert.

Da zwischen Oberteil und Unterteil relativ wenig Platz ist, nachdem sich der Abstand in diesem Bereich beispielsweise zwischen 0,5 cm - 2 cm bewegt, sieht eine zweckmäßige Weiterbildung der Erfindung vor, dass das Füllstück von der Seite her mit seinem zweiten Befestigungsabschnitt in den ersten Befestigungsabschnitt einschiebbar ist. Das heißt, dass das Füllstück seitlich einzuschieben und damit zwischen Ober- und Unterteil bringbar ist. Um ein seitliches Herausrutschen zu verhindern können in Weiterbildung der Erfindung am ersten und am zweiten Befestigungsabschnitt in der Einsetzposition zusammenwirkende Rastelemente, beispielsweise eine Nut und ein Vorsprung und ähnliches, vorgesehen sein. Hierüber wird das Füllstück sicher in seiner seitlichen Ausrichtung fixiert.

Alternativ zur vorstehend beschriebenen Ausführung der beiden Befestigungsabschnitte als komplementär geformte, ineinandergreifende Strukturen sieht eine Erfindungsvariante vor, am Dämpfungselement einen ersten Befestigungsabschnitt und am Füllstück einen zweiten Befestigungsabschnitt vorzusehen, die als Durchbrechungen ausgeführt sind, durch die in der Einsetzposition ein vorzugsweise vom Oberteil her eingeführter Haltestift oder eine ebenfalls vorzugsweise vom Oberteil her eingeführte Halteschraube, die in einen Innengewindeabschnitt, der gegebenenfalls am Dämpfungselement vorgesehen ist, eingreift, greift. Beispielsweise kann am Dämpfungselement wiederum eine Nut vorgesehen sein, die in diesem Fall jedoch nicht hinterschnitten oder sonstwie geformt ist, sondern einen einfachen rechteckigen oder sich nach vorne öffnenden Aufbau aufweist. Von oben ist eine Durchbrechung, also eine Bohrung eingebracht. Eine komplementäre, deckungsgleich zu positionierende Bohrung befindet sich am Füllstück, das beispielsweise wiederum an der Seite eingeschoben wird. Liegen die Bohrungen deckungsgleich, kann von oben durch das Oberteil ein Haltestift oder -bolzen durchgeführt werden, der das Füllstück im Dämpfungselement fixiert. Denkbar ist auch hierzu eine Schraube zu verwenden und beispielsweise in das Dämpfungselement eine Hülse mit einem Innengewinde einzusetzen, in welches die Schraube eingeschraubt wird. Auch hierüber kann eine sichere und vom Träger ohne weiteres lösbare Befestigung des Füllstücks realisiert werden. Die Verformbarkeit des Oberteils darf nicht durch das eingesetzte Halteelement, z. B. die Schraube beeinträchtig werden. Deshalb dient diese nur der Verbindung von Dämpfungselement und Füllstück, sie ist also nicht am Oberteil aufgelagert.

Wenngleich es bereits ausreichend und vorteihaft ist, am Füllstück nur an einem Längsende einen Befestigungsabschnitt vorzusehen, ist es selbstverständlich auch denkbar, das Füllstück an beiden Längsenden mit einem Befestigungsabschnitt auszuführen, so dass das Füllstück in um 180° gedrehter Stellung ebenfalls eingesetzt werden kann. Dies ist insbesondere dann von Vorteil, wenn, worauf nachfolgend noch eingegangen wird, das Füllstück aus unterschiedlichen harten Materialien besteht oder Abschnitte unterschiedlicher Shore-Härte aufweist u. ä.

Eine zweckmäßige Weiterbildung der Erfindung sieht vor, dass das Oberteil und das Unterteil unter Bildung zweier nebeneinander liegender Längsabschnitte von der Fußspitze her längsgeschlitzt ist. Diese Längsschlitzung ermöglicht also die Bildung zweier nebeneinander liegender Federn, die beim Laufen aufgrund der Pronations- oder Supinationsbewegung des Trägers unterschiedlich belastet werden. Erstreckt sich das Füllstück in den Schlitzungsbereich, liegt es also in einem solchen Längsabschnitt, so kann folglich hierüber auch die jeweilige Pronations- oder Supinationsbewegung aufgrund der Wirkung des Füllstücks verändert beziehungsweise eingestellt werden.

Die Breite des Füllstück selbst entspricht im Wesentlichen der Breite des Ober- und Unterteils, jedoch kann das Füllstück auch zumindest abschnittsweise eine geringere Breite als das Ober- und Unterteil aufweisen.

Ist die Fußprothese längsgeschlitzt, sind als zwei nebeneinander liegende Längsabschnitte beziehungsweise Federn gebildet, so sieht eine besonders vorteilhafte Weiterbildung der Erfindung vor, dass das Füllstück zumindest in dem Bereich, mit dem es zwischen zwei übereinander liegenden Längsabschnitten des geschlitzten Ober- und Unterteils angeordnet ist, nur die Breite der Längsabschnitt aufweist. Das heißt, dass das Füllstück so bemessen ist, dass es sich nur in dem Bereich der rechten oder linken Feder, also des rechten oder linken Längsabschnittpaares erstreckt und folglich nur dieser Bereich von der Wirkung des Füllstücks beeinflusst wird. Dies bietet folglich lokal und individuell die Möglichkeit, je nach Art der realen Bewegung die rechte oder linke Feder und damit die jeweilige Pronations- oder Supinationsbewegung über das Füllstück zu beeinflussen. Ein weiterer Vorteil liegt ferner darin, dass in Verbindung der Längsschlitzung und der Möglichkeit der individuellen "seitlichen" Beeinflussung die erfindungsgemäße Fußprothese sowohl am rechten als auch am linken Bein getragen werden kann. Denn durch die Möglichkeit der Integration eines quasi seitlich gesehen nur teilweise respektive abschnittsweise wirkenden Füllstücks kann die Härte so eingestellt werden, dass sie eben der Situation, die beim Tragen am linken oder rechten Bein gegeben ist, entsprechend eingestellt wird. Das heißt, dass das Härte- oder Steifigkeits- respektive Dämpfungsverhalten medial-lateral respektive innen-außen über das Füllstück eingesellt werden kann. Die Lastlinie "wandert" also während der Laufbewegung quasi von einer Seite auf die andere Seite, resultierend aus der nur lokalen Wirkung des Füllstücks.

Um die Variationsmöglichkeit der erfindungsgemäßen Fußprothese noch weiter zu verbessern kann das Füllstück aus mehreren unterschiedliche Shore-Härten aufweisenden Materialien bestehen, oder bei Ausführung aus einem Material Bereiche mit unterschiedlichen Shore-Härten aufweisen. Es besteht also die Möglichkeit, dem Füllstück lokal unterschiedliche Härten respektive Elastizitäten zu verleihen, entweder durch Verwendung verschiedenartiger Materialien, die unterschiedliche Shore-Härten aufweisen, oder durch Verwendung nur eines Materials, das aber lokal unterschiedlich eingestellt ist. Natürlich können auch austauschbare Füllstücke mit unterschiedlichen Shore-Härten zur Verfügung gestellt werden.

Dabei können die Abschnitte aus unterschiedlichen Materialien oder die Bereiche unterschiedlicher Shore-Härte in Breiten- oder in Längsrichtung nebeneinander angeordnet sein. Das heißt, dass unterschiedlich harte Bereiche in Längsrichtung hintereinander liegen, gleichermaßen aber auch in Breitenrichtung variieren können. Je nach Ausgestaltung ändert sich wiederum dementsprechend die Härte des Vorfußbereichs.

Alternativ oder zusätzlich zur Verwendung unterschiedlich harter Materialien oder zur Ausbildung unterschiedlich harter Bereiche besteht die Möglichkeit, dass das Füllstück auch eine über seine Länge- und/oder Breite variierende Dicke aufweist. Das heißt, dass es beispielsweise lateral dicker sein kann als medial oder umgekehrt, wie auch in Längsrichtung hintereinander geschaltete, z. B. wellenförmige Verdickungen realisiert werden können. Über solche Dickenvariationen können die lokalen Berührungsbereiche quasi in Abhängigkeit der Belastung respektive des Fortschreitens der Schrittbewegung sich verändern. Sind beispielsweise die in Längsrichtung hintereinander geschalteten Wellen vorgesehen, so treten während der Aufsetz- oder Abrollbewegung zunächst die Wellenberge in Kontakt mit der jeweiligen Ober- und Unterteilfläche. Bei zunehmendem Fortgang der Bewegung werden die Wellenberge zunehmend komprimiert, die Berührungsflächen vergrößern sich, der Widerstand steigt noch stärker an.

Das Füllstück selbst kann als Vollmaterialbauteil aus einem elastischen Material gebildet sein. Denkbar ist aber auch die Verwendung eines geschäumten elastischen Kunststoffmaterials, das heißt, dass das Füllstück ein Schaumkunststoffbauteil ist. Denkbar ist aber auch die Verwendung eines nicht kompressiblen Füllstücks.

Verwendet werden kann jedweder Kunststoff, der die gewünschte Elastizität beziehungsweise Shore-Härte aufweist und der auch einen hinreichenden Reibungskoeffizienten beziehungsweise Haftfähigkeit besitzt, damit der Widerstand erhöht wird. Denn die Widerstandserhöhung geht letztlich auch mit der Haftfähigkeit des Füllstücks zur jeweiligen Oberteil- und Unterteilfläche einher.

Das Oberteil und das Unterteil selbst sind bevorzugt einstückig miteinander ausgeformt. Handelt es sich bei der Fußprothese um ein Karbon- oder Kohlefaserverbundbauteil, so wird lediglich ein Karbon- oder Kohlestofffaserelement verwendet, das quasi schlaufenförmig verläuft und als einstückiges Bauteil das Unterteil, das im Fußspitzenbereich in das Oberteil übergeht, ausbildet. Alternativ dazu ist es auch denkbar, dass das Oberteil und das Unterteil im Bereich der Fußspitze über ein Verbindungsmittel miteinander verbunden sind. Denkbar ist beispielsweise beide über eine Schraubenverbindung miteinander zu fixieren, wobei bei Ausführung der Prothese als längsgeschlitztes Bauteil jede Feder über ein separates Verbindungsmittel zwischen Oberteil und Unterteil verfügt.

Wie beschrieben besteht für den Träger die Möglichkeit, die Härte seiner Fußprothese beliebig einstellen zu können. Handelt es sich bei dem Füllstück um ein Bauteil aus einem elastischen Kunststoffmaterial, so ist es ohne weiteres möglich, sich die gewünschte Form des Füllstücks individuell durch entsprechendes Zuschneiden des Füllstücks festzulegen. Das heißt, dass der Orthopädietechniker oder der Träger selbst die gewünschte, gegebenenfalls auch asymmetrische Geometrie des Füllstücks definieren kann. Auch bietet sich die Möglichkeit, das Füllstück in seiner Länge zu variieren, wozu zweckmäßigerweise am Füllstück an wenigstens einer Seite Längenangaben vorgesehen sind, die dem Träger entsprechende Informationen geben, wie weit sich beispielsweise das Füllstück in den Vorderfußbereich erstreckt etc. Auch kann hierüber eine entsprechende Anpassung an unterschiedlich lange Fußprothesen erfolgen.

Weitere Vorteile, Merkmale und Einzelheiten der Erfindung ergeben sich aus den im Folgenden beschriebenen Ausführungsbeispielen sowie anhand der Zeichnung. Dabei zeigen:
- Fig. 1: eine Perspektivdarstellung einer erfindungsgemäßen Fußprothese,
- Fig. 2: eine Seitenansicht der Fußprothese aus Fig. 1,
- Fig. 3: eine Aufsicht auf die Fußprothese aus Fig. 1,
- Fig. 4: eine Detailansicht des Dämpfungselements und des Füllstücks in getrennter Stellung,
- Fig. 5: eine vergrößerte Schnittansicht des Verbindungsbereichs des Dämpfungselements mit dem Füllstück,
- Fig. 6: eine Darstellung einer zweiten Ausführungsform des Füllstücks,
- Fig. 7: eine Darstellung einer dritten Ausführungsform des Füllstücks,
- Fig. 8: eine Seitenansicht einer vierten Ausführungsform des Füllstücks, und
- Fig. 9: eine Prinzipdarstellung einer zweiten Befestigungsmöglichkeit des Füllstücks am Dämpfungselement.

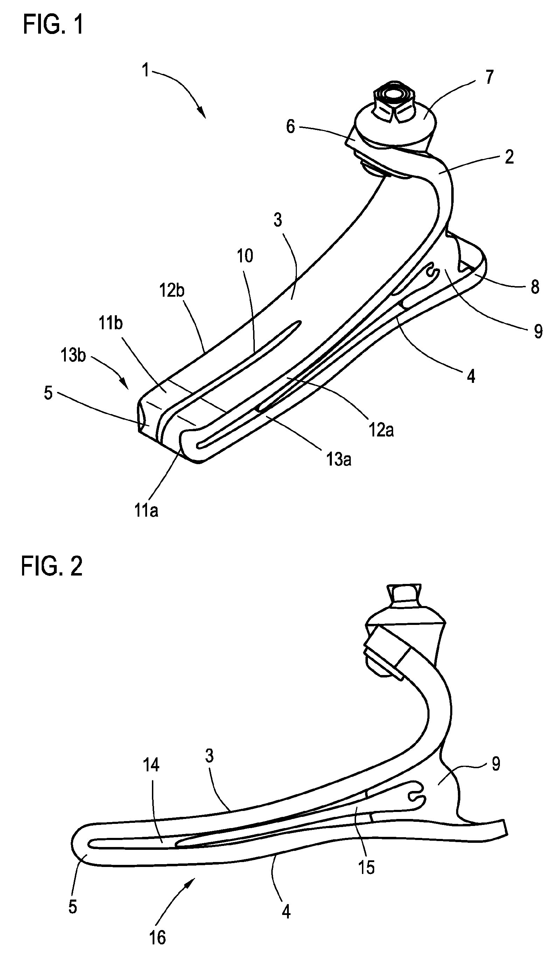

Fig. 1 zeigt eine erfindungsgemäße Fußprothese 1 bestehend aus einem einstückigen Karbon- oder Kohlefaserverbundbauteil 2, das ein Oberteil 3 sowie ein unterhalb des Oberteils 3 geführtes Unterteil 4 aufweist, die im Bereich der Fußspitze 5, da einstückig, miteinander verbunden sind. Am oberen Ende 6 des zu diesem Ende hin gebogenen Oberteils 3 befindet sich ein Anschlussmittel 7 zum Verbinden der Fußprothese mit einem Prothesenhalter. Das Unterteil 4 selbst setzt beim Laufen auf dem Boden auf, das hintere Ende 8 bildet das Fersenteil.

Das Oberteil 3 und das Unterteil 4 sind im Bereich des hinteren Endes 8, also im Fersenbereich über ein Dämpfungselement 9 miteinander verbunden. Dieses Dämpfungselement 9 besteht aus einem elastischen Werkstoff, vornehmlich einem Gummi- oder Kunststoffbauteil, das fest mit Ober- und Unterteil 3, 4 verbunden, vorzugsweise verklebt ist.

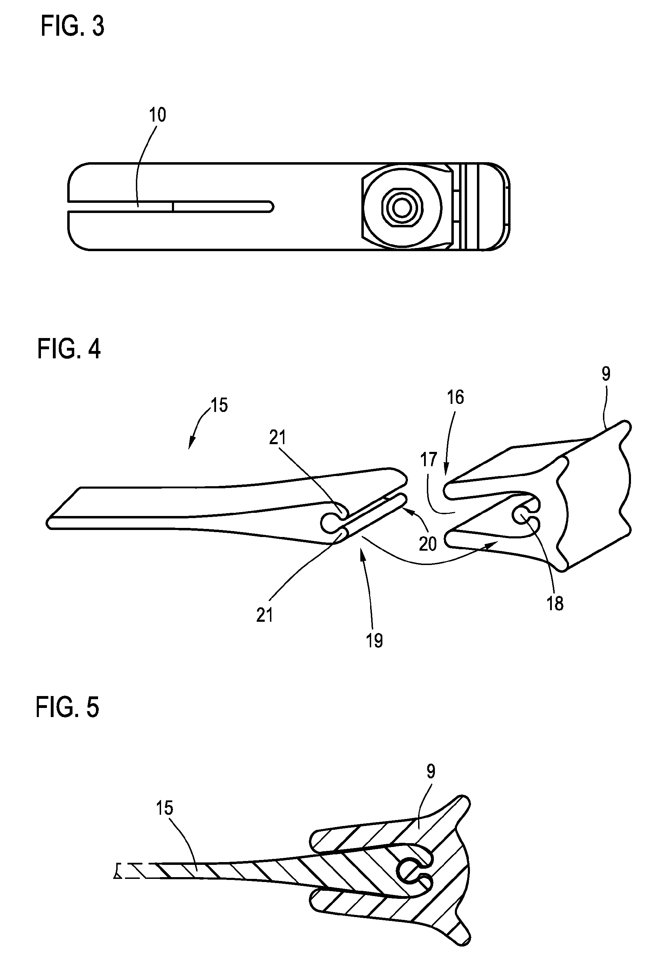

Das Oberteil 3 und das Unterteil 4 sind, siehe Fig. 1 und 3, über einen Teil ihrer Länge über einen Längsschlitz 10 getrennt, so dass sich zwei separate Federn 11 a, 11 b bilden, jeweils bestehend aus zwei Längsabschnitten des Ober- und Unterteils 3, 4, nämlich den Längsabschnitten 12a, 12b des Oberteils 3 und den Längsabschnitten 13a, 13b des Unterteils 4. Das heißt, dass die beiden Federn 11 a, 11 b separat zueinander beweglich sind beziehungsweise separat zueinander einfedern können.

Wie insbesondere Fig. 2 zu entnehmen ist ist zwischen Oberteil 3 und Unterteil 4 ein Hohlraum 14 ausgebildet, in den bei der erfindungsgemäßen Fußprothese ein Füllstück 15 eingesetzt ist. Dieses Füllstück 15 ist als flächiges, längliches und zungenartiges Bauteil ausgeführt. Mit seinem einen Ende ist es am Dämpfungselement 9 befestigt, worauf nachfolgend noch näher eingegangen wird. Es erstreckt sich beispielsweise über näherungsweise 2/3 der Länge des Hohlraums vom Dämpfungselements 9 zur Fußspitze 5 hin. Seine Breite ist derart bemessen, dass sie im Wesentlichen der Breite des Ober- und Unterteils 3, 4, die gleich breit sind, entspricht.

Seine Funktion ist derart, dass es bei einer Laufbewegung beginnend mit dem Aufsetzen des Fersenbereichs 8 des Unterteils 4 bis hin zum Ende des Schritts, wenn sich der Träger mit der Fußspitze 5 abdrückt, temporär nach Erreichen einer bestimmten Laufphase das Oberteil 3 mit dem Unterteil 4 reibungskoppelt, so dass diese über das Füllstück 15 miteinander verbunden sind. Während der Laufbewegung stellt sich eine Relativbewegung von Oberteil 3 zu Unterteil 4 ein, die aufgrund der Gewichtsbelastung der Fußprothese 1 durch das Gewicht des Trägers beim Schreiten einsetzt, insbesondere dann, wenn der Bereich des Vorderfußes 16 auftritt und der Träger beginnt, sich zur Fußspitze 5 hin abzurollen und mit dieser abzudrücken. Hierbei bewegen sich das Ober- und das Unterteil 3, 4, die zunächst beim Auftreten des Fersenbereichs 8 aufgrund der Elastizität des Prothesenmaterials auseinandergespannt werden, wieder aufeinander zu, das heißt, dass die Höhe des Hohlraums 14 abnimmt. Ab einem bestimmten Zeitpunkt liegen die Innenflächen des Ober- und Unterteils 3, 4 am Füllstück 15 an, das aufgrund seiner Materialwahl einen hinreichenden Reibungskoeffizienten besitzt und eine gute Haftung zur jeweiligen anliegenden Fläche bietet. Das Oberteil 3 und Unterteil 4 können sich bei fortschreitender Bewegung nicht mehr ungedämpft relativ zueinander bewegen, sowohl was die Bewegung aufeinander zu als auch die Längsverschiebung zueinander betrifft, wobei diese Längsverschiebung relativ gering ist. Vielmehr ist die Bewegung über das Füllstück 15 gedämpft, das heißt, ihr wird über das Füllstück 15 infolge der Flächenkopplung ein erhöhter Widerstand entgegengesetzt. Hieraus resultiert, dass insgesamt insbesondere der Bereich des Vorderfußes 16, also die Feder, härter wird, mithin also nicht mehr so leicht verformbar ist, da die Biegebewegung in diesem Bereich durch das Füllstück 15 und die Flächenkopplung widerstandsbehaftet ist. Die Fußprothese 1 ist folglich beim Laufen nicht mehr derart "weich" beziehungsweise elastisch, verglichen mit der Prothese ohne eingesetztem Füllstück 15.

Das Füllstück 15 ist lösbar am Dämpfungselement 9 befestigt, kann also vom Träger nach Belieben eingesetzt und entnommen werden. Hierzu ist am Dämpfungselement 9 ein erster Befestigungsabschnitt 16 vorgesehen, bestehend aus einer Nut 17, im Bereich deren hinteren Endes ein beidseitig hinterschnittener, länglicher Haltevorsprung 18, querschnittlich rund, ausgeformt ist.

Am Füllstück 15 ist an seinem hinteren Ende ein zweiter Befestigungsabschnitt 19 ausgebildet, der von der Form her komplementär zur Nut 17 und zum Haltevorsprung 18 ist. Er weist eine der Form des Haltevorsprungs 18 komplementäre Nut 20 auf, die beidseits von klauenartigen Abschnitten 21 umgriffen ist, deren Form wiederum der Nutform um den Haltevorsprung 18 entspricht. Das heißt, dass der zweite Befestigungsabschnitt als eine Halteklaue ausgebildet ist.

Zur Montage wird das Füllstück 15 von der Seite her in die Nut 17 im Dämpfungselement 9 eingeschoben. Wie die vergrößerte Schnittansicht gemäß Fig. 5 zeigt, greifen die beiden Halteabschnitte 16, 19 formschlüssig in der Montagestellung ineinander. Infolge der Anordnung des Haltevorsprungs 18 am Dämpfungselement 9 und der Halteklaue am Füllstück 15 wird verhindert, dass beim Auftreten mit der Ferse, wenn also die Kraft hinter dem Dämpfungselement 9 eingeleitet wird, dieses aufgeht und die Verbindung sich lösen könnte. Denn wenn überhaupt öffnet sich der vordere Bereich der Nut 17 etwas, der Bereich des klauenartigen Umgriffs jedoch nicht, so dass stets unabhängig von der jeweiligen Laufphase eine feste Fixierung gegeben ist.

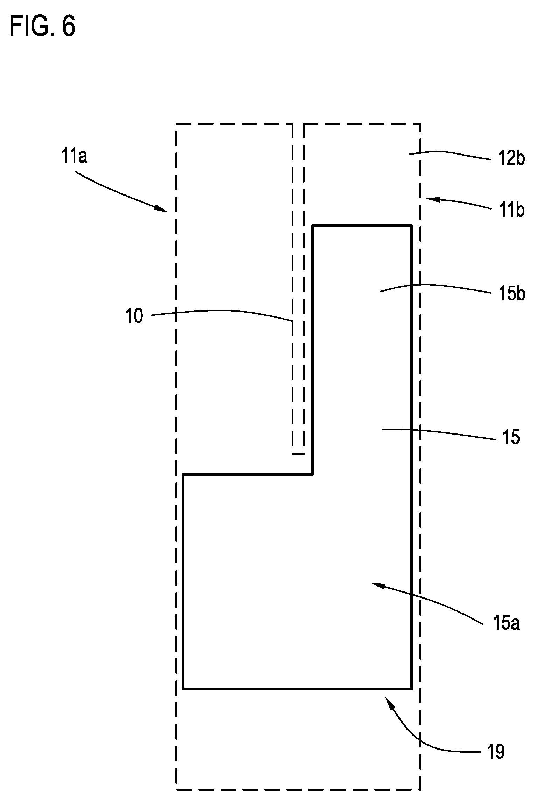

Fig. 6 zeigt eine Prinzipdarstellung einer weiteren Ausführungsform eines Füllstücks 15. Dieses ist asymmetrisch ausgeführt. Mit seinem hinteren Ende, an dem nicht näher gezeigt der zweite Befestigungsabschnitt 19 vorgesehen ist, ist es wiederum im hier nicht näher gezeigten Dämpfungselement 9, das ebenfalls wie in Fig. 4 ausgeführt ist, fixiert. Es erstreckt sich mit seinem breiten Bereich 15a in den Bereich des Vorderfußes 16, in dem also das Ober- und Unterteil 3, 4 bereits übereinander liegen. Der breite Bereich 15a entspricht im Wesentlichen der Breite des Ober- und Unterteils 3, 4. Dieser Abschnitt erstreckt sich im Wesentlichen bis zum Ende des Längsschlitzes 10 des hier nur gestrichelt gezeigten Karbon- oder Kohlefaserverbundbauteils 2. An dem Abschnitt 15a schließt sich ein verlängerter Abschnitt 15b an, der jedoch wesentlich schmäler ist, seine Breite entspricht im Wesentlichen der Breite der beiden übereinander liegenden Längsabschnitte 12b, 13b, also letztlich der Feder 11 b, bezogen auf die Darstellung in Fig. 1. Das heißt, dass hier das Füllstück 15 letztlich das Biegeverhalten im Bereich der rechten Feder 11 b beeinflusst, diese wird infolge der Kopplung der Längsabschnitte 12b, 13b über das Füllstück 15 respektive den Abschnitt 15b härter, verglichen mit der Feder 11a, zwischen die kein Abschnitt des Füllstücks 15 greift. Diese ist folglich gegenüber der Feder 11 b weicher. Dies führt nun dazu, dass je nach Laufstil die Pronation oder Supination beeinflusst werden kann, im Rahmen welcher asymmetrisch die Federn 11 a, 11b infolge einer Fußverdrehung belastet werden. Selbstverständlich besteht die Möglichkeit, das Füllstück 15 auch anders auszugestalten und den verlängerten Abschnitt 15b auf der anderen Seite anzuordnen, so dass dieser in den Bereich der Feder 11 a läuft, die dann beeinflusst wird, während die Feder 11 b unbeeinflusst ist. Folglich kann die Lastkennlinie bezogen auf die Fußlängsachse letztlich variiert werden, indem entweder zwischen beiden Federn 11 a, 11 b ein Füllstück 15 eingebracht wird oder nur in dem Bereich einer Feder. Dies kann vom Träger beliebig eingestellt werden, je nach dem, wie es für ihn komfortabler ist.

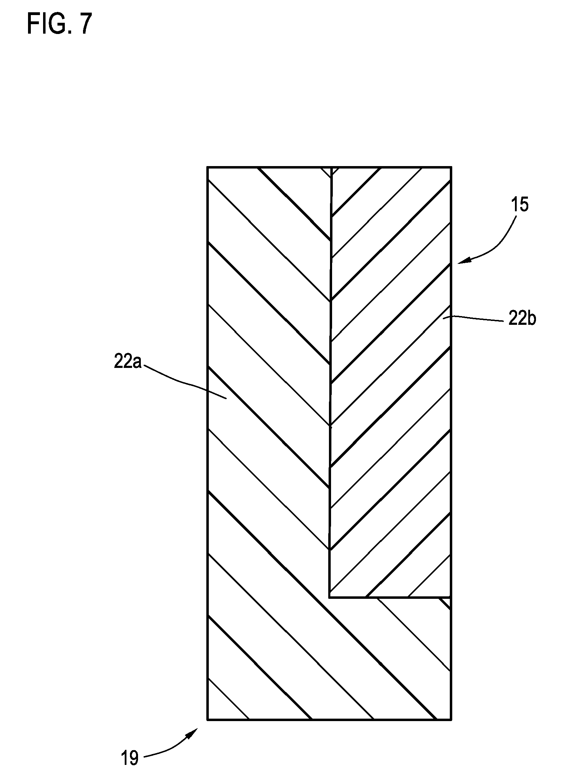

Fig. 7 zeigt eine weitere Ausführungsform eines Füllstücks 15, das hinsichtlich seines zweiten Befestigungsabschnitts 19 wiederum wie in Fig. 4 gezeigt ausgeführt ist. Es weist eine einheitliche Breite über seine Länge auf, jedoch weist es zwei unterschiedlich harte Abschnitte 22a, 22b auf, wie durch die unterschiedliche Strichelung angedeutet ist. Beispielsweise ist der Abschnitt 22b härter als der Abschnitt 22a, das heißt, dass das dortige Material eine höhere Shore-Härte aufweist als im anderen Abschnitt. Dies kann beispielsweise dadurch realisiert werden, dass die Abschnitte 22a, 22b von unterschiedlichen Kunststoffmaterialien gebildet sind, die ohne weiteres in einem gemeinsamen Herstellungsverfahren miteinander verspritzt werden können. Denkbar ist es aber auch, für beide Abschnitte den gleichen Kunststoff zu verwenden, diesen jedoch lokal durch entsprechende Zusätze in seiner Shore-Härte etwas anders einzustellen. In jedem Fall kann über ein solches Element wiederum das Elastizitätsverhalten einer Feder 11 a, 11 b unterschiedlich zu dem der anderen Feder eingestellt werden. Denn die Breite des Abschnitts 22b entspricht im Wesentlichen der Breite einer Feder 11 a, 11 b, im gezeigten Beispiel wäre dies die Feder 11 b, während die Breite des benachbarten schmalen Bereichs des Abschnitts 22a der Breite der Feder 11a entspricht. Die Feder 11 b wäre also anzunehmender Weise härter als die Feder 11 a.

Da der zweite Befestigungsabschnitt 19 symmetrisch aufgebaut ist, besteht die Möglichkeit, das Füllstück 15 auch um 180° gedreht einzusetzen. Das heißt, der Träger kann den im Beispiel angenommener Maßen etwas härteren Abschnitt 22b in der Feder 11 a positionieren, während der weichere Abschnitt 22a in der Feder 11 b positioniert wäre.

Die Abschnitte 22a, 22b können, wenngleich nicht näher gezeigt, auch über einen Längsschlitz voneinander getrennt sein, wobei der Längsschlitz dann deckungsgleich zum Längsschlitz 10 der Prothese verlaufen würde.

Darüber hinaus bietet die Möglichkeit der Ausführung des Füllstücks 15 - und dies gilt für alle beschriebenen Ausführungsformen - aus einem elastischen Material für den Orthopädietechniker oder den Träger die Möglichkeit dieses bei Bedarf mit einer Schere oder dergleichen zurechtzuschneiden. Beispielsweise kann er ein in seinem Verhalten homogenes Füllstück 15, wie in Fig. 4 gezeigt, ohne weiteres zu einer Form zurechtschneiden, wie sie bei dem Füllstück 15 in Fig. 6 gezeigt ist. Auch besteht die Möglichkeit, das Füllstück 15 aus Fig. 7 teilweise dadurch zurechtzuschneiden, dass der eine oder andere Bereich 22a, 22b herausgeschnitten wird, wenn dies komfortabler erscheint.

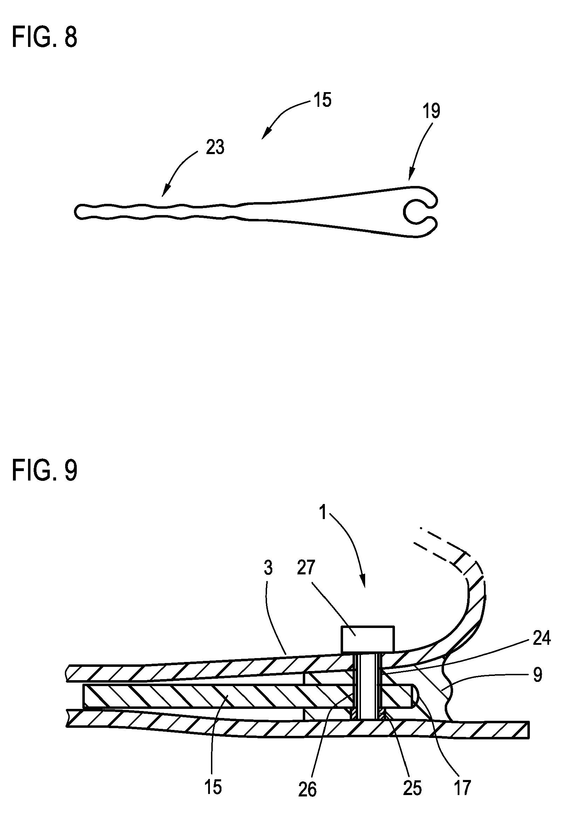

Fig. 8 zeigt eine Seitenansicht einer weiteren Ausführungsform eines Füllstückss 15, das wiederum den zweiten Befestigungsabschnitt 19 aufweist, jedoch keine ebenflächige Form aufweist, sondern ein Wellenprofil 23. Dies führt dazu, dass die Wellenberge zunächst in Kontakt mit den Flächen des Ober- und Unterteils 3, 4 treten. Bei zunehmender Annäherung des Oberteils 3 zum Unterteil 4 werden die Wellenberge zusammengedrückt, die Anlagefläche vergrößert sich zunehmend, der hierüber reibungsbedingt entgegengesetzte Widerstand nimmt überproportional zu. Auch hierüber kann eine Variation der Prothesenhärte erreicht werden. Statt eines Wellenprofils wäre z. B. auch ein Zick-Zack-Profil denkbar.

Fig. 9 zeigt schließlich eine weitere Ausführungsform einer Fußprothese 1 in einer Teilansicht, bei der die Befestigung des Füllstücks 15 in anderer Weise als zuvor beschrieben erfolgt. Am Dämpfungselement 9 ist eine im wesentlichem vertikal verlaufende Bohrung 24 vorgesehen, in die am unteren Ende beispielsweise eine Gewindehülse 25 eingesetzt ist. Das Füllstück 15 weist an seinem hinteren Ende, mit dem es in die auch hier vorgesehene, nicht besonders konturierte Nut 17 eingreift, ebenfalls eine Bohrung 26 auf, die in der Montagestellung mit der Bohrung 24 im Dämpfungselement 9 fluchtet. Von oben, also vom Oberteil 3 herkommend, wird eine Befestigungsschraube 27 eingesetzt, die in der Gewindehülse 25 verschraubt wird. Der Schraubenkopf ruht auf dem Dämpfungselement 9, das Oberteil 3 ist nicht mit der Schraube verbunden. Hierüber wird wiederum eine sichere Fixierung des Füllstücks 15 gewährleistet. Über diese Schraube 27 wird gleichzeitig eine Seitenfixierung realisiert. Bei der Befestigungsweise, wie bezüglich der Figuren 4 ff. beschrieben, kann eine Seitenfixierung dadurch realisiert werden, dass im Bereich der zusammenwirkenden ersten und zweiten Befestigungsabschnitte 16, 19 miteinander in der richtigen Montagestellung zusammenwirkende Rastabschnitte, beispielsweise eine schmale Nut oder Eintiefung und ein entsprechend geformter Vorsprung, zusammenwirken und so eine Seitenfixierung bieten.

Wenngleich in den Figuren ein einteiliges Prothesenbauteil 2 beschrieben ist, besteht selbstverständlich die Möglichkeit, Oberteil 3 und Unterteil 4 als getrennte Bauteile zu fertigen. Diese wären dann im Bereich der Fußspitze 5 über ein Verbindungselement, beispielsweise eine Schraubverbindung oder Ähnliches miteinander zu fixieren. Die Einsatzmöglichkeit eines Füllstücks 15 besteht gleichermaßen bei dieser Ausgestaltung. Sofern auch bei dieser Ausgestaltung ein Längsschlitz zur Bildung zweier separater nebeneinander liegender Federn ausgebildet wird, wären beide Federenden im Bereich der Fußspitze 5 über entsprechende Verbindungsmittel zu verbinden.

## Patentansprüche

1. Fußprothese, umfassend ein Oberteil (3) und ein beim Laufen bodenseitig aufsetzendes Unterteil (4), wobei das Oberteil (3) und das Unterteil (4) über ein im Fersenbereich angeordnetes elastisches Dämpfungselement (9), das am Oberteil (3) und am Unterteil (4) befestigt ist, miteinander verbunden sind und das Oberteil (3) und das Unterteil (4), eine Feder bildend, übereinander und voneinander beabstandet bis in den Bereich der Fußspitze laufen, wobei im Bereich zwischen dem Dämpfungselement (9) und der Fußspitze (5) zwischen das Ober- und das Unterteil (3, 4) wenigstens ein Füllstück (15) eingebracht ist, das beim Laufen in Kontakt mit einer oder mit beiden einander zugewandten Flächen des Ober- und Unterteils (3, 4) tritt, und über welches die Biegesteifigkeit des beim Laufen über das Füllstück gekoppelten Ober- und Unterteils erhöht wird, **dadurch gekennzeichnet, dass** das Füllstück (15) ein flächiges Bauteil mit wenigstens einer anhaftenden Anlagefläche ist.

2. Fußprothese nach Anspruch 1, **dadurch gekennzeichnet, dass** das Füllstück (15) fest mit dem Oberteil (3) oder dem Unterteil (4) verbunden und beim Laufen in Kontakt mit dem Unterteil (4) oder dem Oberteil (3), dabei den Widerstand erzeugend, tritt.

3. Fußprothese nach Anspruch 2, **dadurch gekennzeichnet, dass** das Füllstück (15) mit dem Oberteil (3) oder dem Unterteil (4) verklebt ist.

4. Fußprothese nach Anspruch 1, **dadurch gekennzeichnet, dass** das Füllstück (15) lösbar am Oberteil (3), am Unterteil (4) oder am Dämpfungselement (9) angeordnet ist.

5. Fußprothese nach Anspruch 4, **dadurch gekennzeichnet, dass** am Dämpfungselement (9) ein erster Befestigungsabschnitt (16) und am Füllstück (15) ein zum ersten Befestigungsabschnitt (16) komplementär geformter zweiter Befestigungsabschnitt (19) vorgesehen ist, die lösbar miteinander in Eingriff bringbar sind.

6. Fußprothese nach Anspruch 5, **dadurch gekennzeichnet, dass** der erste Befestigungsabschnitt (16) eine zur Fußspitze (5) hin offene Nut (17) mit einem innenliegenden hinterschnittenen länglichen Haltevorsprung (18) und der zweite Befestigungsabschnitt (19) eine komplementär zur Nut (17) und zum Haltevorsprung geformt Halteklaue (20), die den Haltevorsprung (18) umgreift, ist.

7. Fußprothese nach Anspruch 6, **dadurch gekennzeichnet, dass** das Füllstück (15) von der Seite her mit seinem zweiten Befestigungsabschnitt (19) in den ersten Befestigungsabschnitt (16) einschiebbar ist.

8. Fußprothese nach Anspruch 6 oder 7, **dadurch gekennzeichnet, dass** am ersten und am zweiten Befestigungsabschnitt (16, 19) ein seitliches Verrutschen verhindernde, in der Einsetzposition zusammenwirkende Rastelemente vorgesehen sind.

9. Fußprothese nach Anspruch, 4, **dadurch gekennzeichnet, dass** am Dämpfungselement (9) ein erster Befestigungsabschnitt und am Füllstück (15) ein zweiter Befestigungsabschnitt vorgesehen ist, die als Durchbrechungen (24, 26) ausgeführt sind, durch die in der Einsetzposition ein vorzugsweise vom Oberteil (3) her eingeführter Haltestift oder eine Halteschraube (27), die in einen Innengewindeabschnitt, der gegebenenfalls am Dämpfungselement (9) vorgesehen ist, eingreift, greift.

10. Fußprothese nach einem der Ansprüche 5 bis 9, **dadurch gekennzeichnet, dass** das Füllstück (15) an beiden Längsenden einen zweiten Befestigungsabschnitt aufweist.

11. Fußprothese nach einem der vorangehenden Ansprüche, **dadurch gekennzeichnet, dass** das Oberteil (3) und das Unterteil (4) unter Bildung zweier nebeneinander liegender Längsabschnitte (12a, 12b, 13a, 13b) von der Fußspitze (5) her längsgeschlitzt ist.

12. Fußprothese nach einem der vorangehenden Ansprüche, **dadurch gekennzeichnet, dass** die Breite des Füllstücks (15) im wesentlichen der Breite des Ober- und Unterteils (3, 4) entspricht, oder dass das Füllstück (15) zumindest abschnittsweise eine geringere Breite als das Ober- und das Unterteil (3, 4) aufweist.

13. Fußprothese nach Anspruch 11 und 12, **dadurch gekennzeichnet, dass** das Füllstück (15) zumindest im Bereich (15b), mit dem es zwischen zwei übereinander liegenden Längsabschnitten (12a, 13a, 12b, 13b) des geschlitzten Ober- und Unterteils (3, 4) angeordnet ist, nur im wesentlichen die Breite der Längsabschnitte (12a, 13a, 12b, 13b) aufweist.

14. Fußprothese nach einem der vorangehenden Ansprüche, **dadurch gekennzeichnet, dass** Füllstücke (15) mit unterschiedlichen Shore-Härten einsetzbar sind.

15. Fußprothese nach einem der vorangehenden Ansprüche, **dadurch gekennzeichnet, dass** das Füllstück (15) aus mehreren unterschiedliche Shore-Härten aufweisenden Materialien besteht, oder mehrere unterschiedliche Shore-Härten aufweisende Bereiche (22a, 22b) aufweist.

16. Fußprothese nach Anspruch 15, **dadurch gekennzeichnet, dass** die Abschnitte (22a, 22b) aus unterschiedlichen Materialien oder die Bereiche unterschiedlicher Shore-Härte in Breiten- oder in Längsrichtung nebeneinander angeordnet sind.

17. Fußprothese nach einem der vorangehenden Ansprüche, **dadurch gekennzeichnet, dass** das Füllstück (15) eine über seine Länge und/oder Breite variierende Dicke aufweist.

18. Fußprothese nach einem der vorangehenden Ansprüche, **dadurch gekennzeichnet, dass** das Füllstück (15) als Vollmaterialbauteil aus einem elastischen Kunststoffmaterial gebildet ist, oder dass es als Schaummaterialbauteil aus einem geschäumten elastischen Kunststoffmaterial gebildet ist.

19. Fußprothese nach einem der vorangehenden Ansprüche, **dadurch gekennzeichnet, dass** das Oberteil (3) und das Unterteil (4) einstückig miteinander ausgeformt sind.

20. Fußprothese nach einem der Ansprüche 1 bis 18, **dadurch gekennzeichnet, dass** das Oberteil (3) und das Unterteil (4) im Bereich der Fußspitze (5) über ein Verbindungsmittel miteinander verbunden sind.

21. Fußprothese nach einem der vorangehenden Ansprüche, **dadurch gekennzeichnet, dass** am Füllstück (15) an wenigstens einer Seite Längenangaben vorgesehen sind.

## Claims

1. Foot prosthesis, comprising an upper part (3), and a lower part (4) that is placed on the ground when walking, wherein the upper part (3) and the lower part (4) are connected to each other via an elastic damping element (9) which is fixed on the upper part (3) and on the lower part (4) and is arranged in the heel area, and the upper part (3) and the lower part (4), forming a spring, extend one above the other and spaced apart from each other as far as the area of the forefoot, wherein at least one filling piece (15) is inserted between the upper part (3) and the lower part (4) in the area between the damping element (9) and the forefoot (5), which filling piece (15), during walking, comes into contact with one or with both mutually facing surfaces of the upper and lower part (3,4), and which filling piece (15) increases the flexural stiffness of the upper and lower part coupled via the filling piece during walking, **characterized in that** the filling piece (15) is a planar component with at least one adhesive contact surface.

2. Foot prosthesis according to Claim 1, **characterized in that** the filling piece (15) is fixedly connected to the upper part (3) or the lower part (4) and, during walking, comes into contact with the lower part (4) or the upper part (3), thus generating the resistance.

3. Foot prosthesis according to Claim 2, **characterized in that** the filling piece (15) is glued to the upper part (3) or to the lower part (4).

4. Foot prosthesis according to Claim 1, **characterized in that** the filling piece (15) is arranged releasably on the upper part (3), on the lower part (4) or on the damping element (9).

5. Foot prosthesis according to Claim 4, **characterized in that** a first securing portion (16) is provided on the damping element (9), and a second securing portion (19) complementing the first securing portion (16) is provided on the filling piece (15), and these securing portions can be brought into releasable engagement with each other.

6. Foot prosthesis according to Claim 5, **characterized in that** the first securing portion (16) is a groove (17), which is open toward the forefoot (5) and has an undercut and elongate holding projection (18) in the inside, and the second securing portion (19) is a holding claw (20), which is shaped to complement the groove (17) and the holding projection and engages around the holding projection (18).

7. Foot prosthesis according to Claim 6, **characterized in that** the filling piece (15) can be pushed with its second securing portion (19) sideways into the first securing portion (16).

8. Foot prosthesis according to Claim 6 or 7, **characterized in that** locking elements provided on the first and second securing portions (16, 19) cooperate in the insertion position and prevent lateral slipping.

9. Foot prosthesis according to Claim 4, **characterized in that** a first securing portion is provided on the damping element (9) and a second securing portion is provided on the filling piece (15), said securing portions being designed as apertures (24, 26) which, in the insertion position, provide a passage for a holding pin, preferably introduced from the direction of the upper part (3), or a holding screw (27), which engages in an internally threaded portion optionally provided on the damping element (9).

10. Foot prosthesis according to one of Claims 5 to 9, **characterized in that** the filling piece (15) has a second securing portion at both longitudinal ends.

11. Foot prosthesis according to one of the preceding claims, **characterized in that** the upper part (3) and the lower part (4) are longitudinally slit from the direction of the forefoot (5) so as to form two adjacent longitudinal portions (12a, 12b, 13a, 13b).

12. Foot prosthesis according to one of the preceding claims, **characterized in that** the width of the filling piece (15) corresponds substantially to the width of the upper part (3) and lower part (4), or **in that** the filling piece (15) has at least in some areas a smaller width than the upper part (3) and lower part (4).

13. Foot prosthesis according to Claims 11 and 12, **characterized in that** the filling piece (15), at least in the area (15b) with which it is arranged between two mutually superposed longitudinal portions (12a, 13a, 12b, 13b) of the slotted upper part (3) and lower part (4), has substantially only the width of the longitudinal portions (12a, 13a, 12b, 13b).

14. Foot prosthesis according to one of the preceding claims, **characterized in that** filling pieces (15) with different Shore hardnesses can be used.

15. Foot prosthesis according to one of the preceding claims, **characterized in that** the filling piece (15) is made of several materials having different Shore hardnesses or has several areas (22a, 22b) having different Shore hardnesses.

16. Foot prosthesis according to Claim 15, **characterized in that** the portions (22a, 22b) made of different materials or the areas of different Shore hardness are arranged alongside each other in the transverse direction or in the longitudinal direction.

17. Foot prosthesis according to one of the preceding claims, **characterized in that** the filling piece (15) has a thickness varying over its length and/or width.

18. Foot prosthesis according to one of the preceding claims, **characterized in that** the filling piece (15) is formed as a solid material component made of an elastic plastic, or **in that** it is formed as a foamed material component made of a foamed elastic plastic.

19. Foot prosthesis according to one of the preceding claims, **characterized in that** the upper part (3) and the lower part (4) are formed integrally with each other.

20. Foot prosthesis according to one of Claims 1 to 18, **characterized in that** the upper part (3) and the lower part (4) are connected to each other in the area of the forefoot (5) by a connecting means.

21. Foot prosthesis according to one of the preceding claims, **characterized in that** length markings are provided on at least one side of the filling piece (15)

## Revendications

1. Prothèse du pied, comprenant une partie supérieure (3) et une partie inférieure (4) se posant au sol lors de la course, la partie supérieure (3) et la partie inférieure (4) étant connectées l'une à l'autre par le biais d'un élément d'amortissement élastique (9) disposé dans la région du talon, qui est fixé à la partie supérieure (3) et à la partie inférieure (4), et la partie supérieure (3) et la partie inférieure (4), en formant un ressort, s'étendant l'une au-dessus de l'autre et à distance l'une de l'autre jusque dans la région de la pointe du pied, au moins une pièce de remplissage (15) étant introduite dans la région entre l'élément d'amortissement (9) et la pointe du pied (5) entre la partie supérieure et la partie inférieure (3, 4), laquelle pièce de remplissage, lors de la course, entrant en contact avec une surface ou avec deux surfaces tournées l'une vers l'autre de la partie supérieure et de la partie inférieure (3, 4), et par le biais de laquelle la rigidité en flexion de la partie supérieure et de la partie inférieure accouplées par le biais de la pièce de remplissage lors de le course est accrue, **caractérisée en ce que** la pièce de remplissage (15) est un composant plat ayant au moins une surface d'appui adhérente.

2. Prothèse du pied selon la revendication 1, **caractérisée en ce que** la pièce de remplissage (15) est connectée fixement à la partie supérieure (3) ou à la partie inférieure (4) et lors de la course, vient en contact avec la partie inférieure (4) ou la partie supérieure (3), en produisant ainsi la résistance.

3. Prothèse du pied selon la revendication 2, **caractérisée en ce que** la pièce de remplissage (15) est collée à la partie supérieure (3) ou à la partie inférieure (4).

4. Prothèse du pied selon la revendication 1, **caractérisée en ce que** la pièce de remplissage (15) est disposée de manière amovible sur la partie supérieure (3), sur la partie inférieure (4) ou sur l'élément d'amortissement (9).

5. Prothèse du pied selon la revendication 4, **caractérisée en ce qu'**une première partie de fixation (16) est prévue sur l'élément d'amortissement (9) et une deuxième partie de fixation (19) de forme complémentaire à la première partie de fixation (16) est prévue sur la pièce de remplissage (15), lesquelles peuvent être amenées en prise l'une avec l'autre de manière détachable.

6. Prothèse du pied selon la revendication 5, **caractérisée en ce que** la première partie de fixation (16) est une rainure (17) ouverte vers la pointe du pied (5) avec une saillie de retenue allongée en contre-dépouille située à l'intérieur (18) et la deuxième partie de fixation (19) est une griffe de retenue (20) formée de manière complémentaire à la rainure (17) et à la saillie de retenue, laquelle vient en prise autour de la saillie de retenue (18).

7. Prothèse du pied selon la revendication 6, **caractérisée en ce que** la pièce de remplissage (15) peut être insérée depuis le côté avec sa deuxième partie de fixation (19) dans la première partie de fixation (16).

8. Prothèse du pied selon la revendication 6 ou 7, **caractérisée en ce que** des éléments d'encliquetage coopérant dans la position d'insertion, empêchant un glissement latéral, sont prévues au niveau de la première et de la deuxième partie de fixation (16, 19).

9. Prothèse du pied selon la revendication 4, **caractérisée en ce qu'**une première partie de fixation est prévue sur l'élément d'amortissement (9) et une deuxième partie de fixation est prévue sur la pièce de remplissage (15), lesquelles sont réalisées sous forme d'orifices (24, 26) à travers lesquels, dans la position d'insertion, s'engage une goupille de retenue introduite de préférence depuis la partie supérieure (3) ou une vis de retenue (27), qui vient en prise dans une partie de filetage interne éventuellement prévue au niveau de l'élément d'amortissement (9).

10. Prothèse du pied selon l'une quelconque des revendications 5 à 9, **caractérisée en ce que** la pièce de remplissage (15) présente, au deux extrémités longitudinales, une deuxième partie de fixation.

11. Prothèse du pied selon l'une quelconque des revendications précédentes, **caractérisée en ce que** la partie supérieure (3) et la partie inférieure (4) sont fendues longitudinalement depuis la pointe du pied (5) en formant deux parties longitudinales juxtaposées (12a, 12b, 13a, 13b).

12. Prothèse du pied selon l'une quelconque des revendications précédentes, **caractérisée en ce que** la largeur de la pièce de remplissage (15) correspond essentiellement à la largeur de la partie supérieure et de la partie inférieure (3, 4), ou **en ce que** la pièce de remplissage (15) présente au moins en partie une largeur inférieure à celle de la partie supérieure et de la partie inférieure (3, 4).

13. Prothèse du pied selon les revendications 11 et 12, **caractérisée en ce que** la pièce de remplissage (15), au moins dans la région (15b) avec laquelle elle est disposée entre deux parties longitudinales disposées l'une au-dessus de l'autre (12a, 13a, 12b, 13b) de la partie supérieure et de la partie inférieure (3, 4) fendues, présente seulement essentiellement la largeur des parties longitudinales (12a, 13a, 12b, 13b).

14. Prothèse du pied selon l'une quelconque des revendications précédentes, **caractérisée en ce que** l'on peut utiliser des pièces de remplissage (15) ayant des duretés Shore différentes.

15. Prothèse du pied selon l'une quelconque des revendications précédentes, **caractérisée en ce que** la pièce de remplissage (15) se compose de plusieurs matériaux présentant des duretés Shore différentes ou présente plusieurs régions (22a, 22b) présentant des duretés Shore différentes.

16. Prothèse du pied selon la revendication 15, **caractérisée en ce que** les parties (22a, 22b) constituées de matériaux différents ou les régions de dureté Shore différentes sont disposées les unes à côté des autres dans la direction en largeur ou dans la direction longitudinale.

17. Prothèse du pied selon l'une quelconque des revendications précédentes, **caractérisée en ce que** la pièce de remplissage (15) présente une épaisseur variable sur sa longueur et/ou sur sa largeur.

18. Prothèse du pied selon l'une quelconque des revendications précédentes, **caractérisée en ce que** la pièce de remplissage (15) est formée en tant que composant en matériau massif d'un matériau en plastique élastique ou **en ce qu'**elle est formée en tant que composant en matériau de mousse d'un matériau en plastique élastique moussé.

19. Prothèse du pied selon l'une quelconque des revendications précédentes, **caractérisée en ce que** la partie supérieure (3) et la partie inférieure (4) sont formées d'une seule pièce l'une avec l'autre.

20. Prothèse du pied selon l'une quelconque des revendications 1 à 18, **caractérisée en ce que** la partie supérieure (3) et la partie inférieure (4) sont connectées l'une à l'autre dans la région de la pointe du pied (5) par le biais d'un moyen de connexion.

21. Prothèse du pied selon l'une quelconque des revendications précédentes, **caractérisée en ce que** des indications de longueur sont prévues au moins d'un côté sur la pièce de remplissage (15).
